# FASCICULE DE BREVET EUROPEEN

(11) **EP 0 549 498 B1**
(45) Date de publication et mention de la délivrance du brevet: **09.08.1995**
(21) Numéro de dépôt: 92420483.7
(22) Date de dépôt: 23.12.1992
(51) Int. Cl.: A61M 39/00, E05B 35/00

(54) **Rampe de perfusion**
Infusionsverteiler
Infusion manifold

(30) Priorité: 27.12.1991 FR 9116451
(43) Date de publication de la demande: 30.06.1993
(73) Titulaire: CAIR L.G.L., F-69170 Tarare (FR)
(72) Inventeur: Lopez, Georges Antoine, F-69290 Craponne (FR)
(74) Mandataire: Maureau, Philippe

(56) Documents cités:
- EP-A- 0 453 378
- FR-A- 2 617 717

## Description

La présente invention concerne une rampe de perfusion.

Une telle rampe est utilisée lorsqu'un patient doit recevoir sous perfusion, simultanément ou non, plusieurs produits de nature différente. Elle comprend un certain nombre de robinets à boisseau, généralement trois ou quatre, dont chacun est relié, d'une part, à un récipient contenant l'un des produits à perfuser et, d'autre part, soit directement soit par l'intermédiaire des autres robinets, au tuyau d'acheminement des produits vers le patient.

Ce genre de rampe doit à la fois être parfaitement protégée contre toute contamination microbienne et permettre une manipulation fréquente des robinets, avec toutes garanties de sécurité quant à la manipulation et au positionnement des robinets.

Pour atteindre ces objectifs contradictoires, il est connu d'envelopper les rampes dans un matériau cellulaire souple imbibé de liquide antiseptique et de placer l'ensemble dans un boîtier pouvant être ouvert pour permettre l'accès aux robinets. Le boîtier comporte des moyens pour sa fixation en hauteur, à un mât ou à une potence.

On conçoît aisément que la nécessité d'ouvrir le boîtier et de manipuler les robinets affecte la stérilité de la rampe. De plus, les rampes existantes ne permettent pas de contrôler, sans ouvrir le boîtier, la position des boisseaux des différents robinets. Enfin, le fait de devoir retirer la partie supérieure du matériau cellulaire pour découvrir les organes de manoeuvre des robinets amène l'infirmière ou le patient à se tâcher les doigts, les liquides antiseptiques utilisés étant généralement colorés.

On connaît également par le brevet français N° 2 617 717 une rampe de perfusion de ce type, dont les robinets présentent des organes de manoeuvre qui dépassent à l'extérieur du boîtier, ces organes de manoeuvre étant montés de manière définitive sur les robinets ou sur la coquille supérieure du boîtier.

La forme à trois branches perpendiculaires deux à deux des organes de manoeuvre permet de visualiser immédiatement dans quelle position se trouve le robinet, chaque branche étant disposée parallèlement à l'un des conduits du boisseau du robinet.

Ce dispositif assure une bonne protection de la rampe contre les microbes mais présente l'inconvénient de rendre possible un actionnement non désiré ou une manipulation non autorisée des robinets, ce qui peut avoir des conséquences dommageables pour le patient.

La présente invention telle que caractérisée dans la revendication 1 vise à remédier à l'ensemble de ces inconvénients.

La rampe qu'elle concerne est du type comportant plusieurs robinets à boisseau enveloppés dans un matériau cellulaire souple imbibé d'un liquide antiseptique, contenus dans un boîtier.

Selon l'invention, chaque organe de manoeuvre des robinets est amovible et peut être solidarisé en rotation à la tête du boisseau d'un robinet grâce à une partie mâle profilée pouvant être engagée, au travers d'ouvertures aménagées dans le boîtier et dans le matériau cellulaire en regard du boisseau de chaque robinet, dans une cavité femelle de profil correspondant, des moyens de repérage de la position du boisseau, visibles de l'extérieur du boîtier lorsque ledit organe de manoeuvre est retiré, étant, en outre, prévus.

Ainsi, dans la rampe selon l'invention, les organes de manoeuvre des robinets sont situés à l'extérieur du boîtier, de sorte que les boisseaux des robinets peuvent être actionnés sans qu'il soit nécessaire d'ouvrir le boîtier et de manipuler le matériau cellulaire. La rampe reste ainsi parfaitement stérile et protégée contre la prolifération microbienne. L'amovibilité des organes de manoeuvre interdit toute manipulation intempestive des robinets.

De plus, les moyens de repérage que comprend la rampe sont visibles de l'extérieur du boîtier et renseignent immédiatement sur la position des boisseaux sans qu'il soit nécessaire d'ouvrir le boîtier.

De préférence, les moyens de repérage sont constitués, pour chaque robinet, par une bague qui est engagée sur l'ouverture que comprend le boîtier en regard du robinet et qui est montée pivotante par rapport au boîtier, dont l'ouverture a un profil complémentaire de celui de la partie mâle profilée de l'organe de manoeuvre amovible afin de pouvoir être solidarisée en rotation audit organe de manoeuvre, cette bague comprenant au moins un point de repérage indicatif de la position du boisseau, et le boîtier comprenant des points de repère disposés en correspondance des différentes positions possibles des boisseaux.

L'organe de manoeuvre de chaque robinet fait donc pivoter ladite bague en même temps que le boisseau et la position du ou des points de repère de la bague par rapport aux points de repère du boîtier permet de parfaitement visualiser, depuis l'extérieur du boîtier et après retrait des organes de manoeuvre, la position des boisseaux.

Avantageusement, l'ouverture de la bague présente un profil comprenant au moins une extrémité distincte de nature à constituer le ou les points de repère indicatifs de la position du boisseau, que comprend la bague. Ainsi, en cas de robinet à trois voies, l'ouverture de la bague, et donc par conséquent la section transversale de la cavité femelle du boisseau et de la partie mâle correspondante de l'organe de manoeuvre, peut être de forme triangulaire, chaque angle du triangle correspondant à l'un des conduits du boisseau.

Selon une première forme de réalisation de l'invention, la partie mâle profilée de l'organe de manoeuvre traverse l'ouverture de la bague et vient s'engager dans une cavité femelle aménagée dans le boisseau.

Selon une deuxième forme de réalisation de l'invention, qui, par rapport à la forme de réalisation précitée, présente l'avantage de garantir encore mieux l'aseptie de la rampe, pour chaque robinet, la bague comprend un prolongement axial faisant saillie vers l'intérieur du boîtier, ce prolongement fermant l'ouverture de la bague et ayant un profil mâle complémentaire d'une cavité femelle aménagée dans le boisseau, dans laquelle il est engagé. Il n'y a ainsi aucun contact direct entre le boisseau et l'organe de manoeuvre et donc aucun risque de contamination.

De toute façon, l'invention sera bien comprise à l'aide de la description qui suit, en référence au dessin schématique annexé représentant, à titre d'exemple non limitatif, une forme de réalisation préférée de la rampe de perfusion qu'elle concerne.
Figure 1 en est une vue en perspective avec arrachement partiel ;
Figure 2 en est une vue en coupe selon II-II de figure 1 et selon une première forme de réalisation ;
Figure 3 en est une vue en coupe longitudinale, selon III-III de figure 1 ; et
Figure 4 en est une vue en coupe similaire à la figure 2, selon une deuxième forme de réalisation.

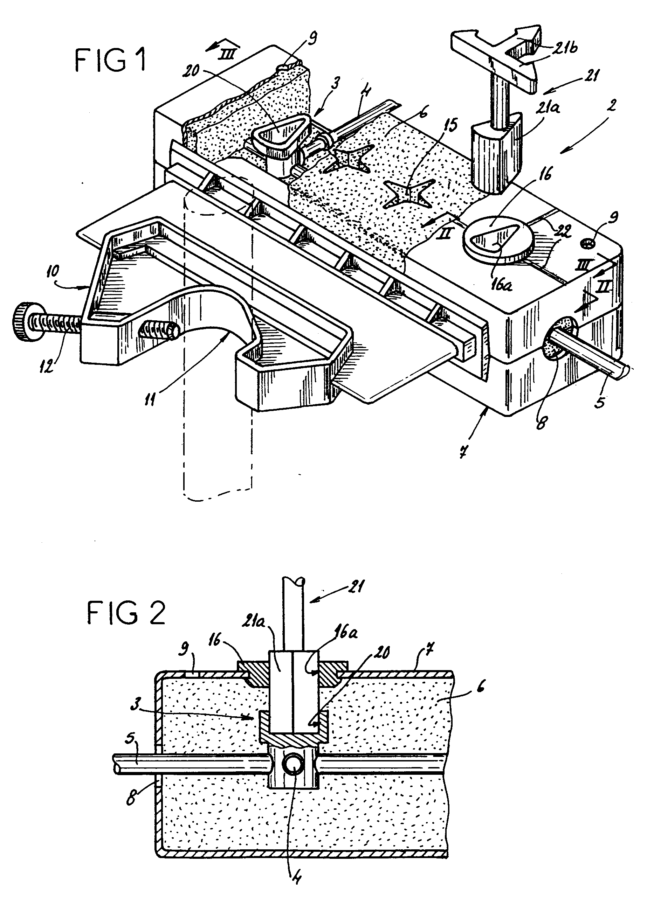

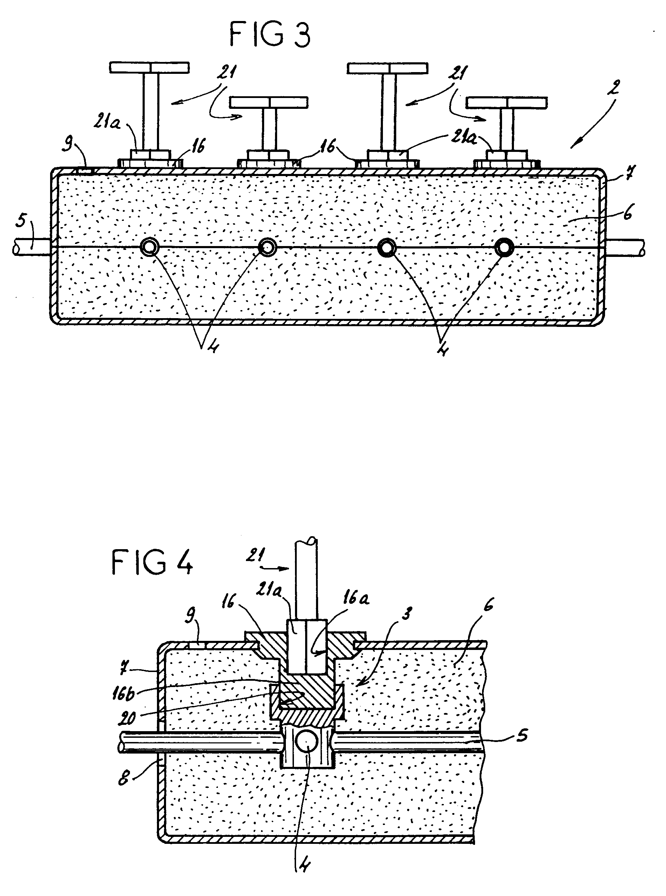

Les figures 1 et 3 représentent sous différents angles une rampe 2 de perfusion comportant plusieurs robinets 3 à boisseau, dont un seul est visible sur la figure 1. Chaque robinet 3 est relié, d'une part, par l'intermédiaire d'un tuyau 4, à un récipient (non représenté) comprenant l'un des produits à perfuser et, d'autre part, directement ou par l'intermédiaire des autres robinets 3, au tuyau 5 d'acheminement des produits à perfuser vers le patient. Les robinets 3, ainsi que l'extrémité des tuyaux 4 et 5, sont enveloppés dans un matériau cellulaire souple 6 imbibé d'un liquide antiseptique, et l'ensemble est contenu dans un boîtier 7. Le boîtier 7 est percé d'orifices 8 pour le passage des tuyaux 4 et 5 et d'orifices 9 pour l'introduction du liquide antiseptique. Il comprend en outre une platine 10 pour son accrochage en hauteur, comprenant une partie concave 11 destinée à être engagée autour d'un mât ou d'une potence, et une vis 12 pour le serrage dudit mât ou de ladite potence contre ladite partie concave 11.

Comme cela est visible sur la figure 1, le boîtier 7 et le matériau cellulaire 6 comprennent des ouvertures ménagées en regard du boisseau de chaque robinet 3. Les ouvertures 15 ménagées dans le matériau cellulaire 6 sont constituées par deux fentes ménagées en croix tandis que les ouvertures ménagées dans le boîtier 7 ont une section circulaire et reçoivent chacune une bague 16 pouvant pivoter par rapport au boîtier 7.

La figure 1 montre également que la tête du boisseau de chaque robinet 3 comprend une cavité femelle 20 présentant un profil de forme sensiblement triangulaire. L'ouverture 16a de chaque bague 16 présente un profil identique à celui des cavités femelles 20 et chaque organe de manoeuvre 21 de chaque robinet 3 est amovible et présente une partie mâle 21a présentant un profil complémentaire de celui de la cavité 20 et de l'ouverture 16a.

Il doit être précisé que les robinets 3 sont à trois voies et que chaque angle du triangle que délimite l'ouverture 16a correspond à l'une des ouvertures du boisseau.

En outre, la face supérieure du boîtier 7, qui comprend les ouvertures aménagées en regard de chaque robinet 3, comporte des points de repère 22 imprimés ou graves sur elle en correspondance des différentes positions possibles des boisseaux.

Il apparaît sur la figure 2 que la partie mâle profilée 21a de l'organe de manoeuvre 21 est destinée à être engagée au travers de l'ouverture 16a de la bague 16 puis dans la cavité 20 de la tête du boisseau du robinet 3. La liaison en rotation obtenue entre l'organe de manoeuvre 21 et le boisseau permet la manoeuvre du robinet 3. L'organe de manoeuvre 21 étant situé à l'extérieur du boîtier 7, les boisseaux des robinets 3 peuvent être actionnés sans qu'il soit nécessaire d'ouvrir le boîtier 7 et de manipuler le matériau cellulaire 6. La rampe 2 reste ainsi parfaitement stérile. L'amovibilité des organes de manoeuvre 21 interdit de plus toute manipulation intempestive, c'est-à-dire non désirée ou non autorisée des robinets 3.

La liaison en rotation entre l'organe de manoeuvre 21 et la bague 16 permet de faire pivoter ladite bague 16 en même temps que le boisseau et d'amener les points de repère de la position du boisseau que forment les angles du triangle que délimite son ouverture 16a en face des points de repère 22. Il est ainsi possible de repérer immédiatement la position des boisseaux depuis l'extérieur du boîtier 7, sans nécessiter d'ouvrir celui-ci.

Bien entendu, le profil de la cavité 20, de la partie mâle 21a et de l'ouverture 16a peut être de formes différentes. Le repérage de la position du boisseau peut alors être simplement réalisé à l'aide des branches perpendiculaires 21b de l'organe de manoeuvre 21. Ceci implique toutefois d'engager l'organe de manoeuvre au travers de l'ouverture 16a pour pouvoir réaliser ce repérage.

La figure 3 montre plus spécifiquement que les organes de manoeuvre 21 peuvent être de longueur différente afin de faciliter leur manipulation.

La figure 4, quant à elle, montre une variante de réalisation de l'invention présentant l'avantage par rapport à celle représentée sur la figure 2 de garantir encore mieux l'aseptie de la rampe 2. Pour chaque robinet 3, la bague 16 comprend un prolongement axial 16b faisant saillie vers l'intérieur du boîtier 7, ce prolongement 16b fermant l'ouverture 16a de la bague 16 et ayant un profil mâle complémentaire de la cavité femelle 20 du boisseau. Ce profil 16b est destiné à être engagé dans la cavité 20. Il n'y a ainsi aucun contact direct entre le boisseau et l'organe de manoeuvre 21 et donc aucun risque de contamination.

## Revendications

1. Rampe de perfusion, du type comportant plusieurs robinets à boisseau, enveloppés dans un matériau cellulaire souple imbibé d'un liquide antiseptique et contenus dans un boîtier, caractérisée en ce que chaque organe de manoeuvre (21) des robinets (3) est amovible et peut être solidarisé en rotation à la tête du boisseau d'un robinet (3) grâce à une partie mâle profilée (16b,21a) pouvant être engagée, au travers d'ouvertures (15) aménagées dans le boîtier (7) et dans le matériau cellulaire (6) en regard des boisseaux des robinets (3), dans une cavité femelle (20) de profil correspondant, des moyens de repérage de la position du boisseau, visibles de l'extérieur du boîtier (7) lorsque ledit organe de manoeuvre (21) est retiré, étant, en outre, prévus.

2. Rampe de perfusion selon la revendication 1, caractérisée en ce que les moyens de repérage sont constitués, d'une part, pour chaque robinet (3), par une bague (16) qui est engagée sur l'ouverture que comprend le boîtier (7) en regard du robinet (3) et qui est montée pivotante par rapport au boîtier (7), dont l'ouverture (16a) a un profil complémentaire de celui de la partie mâle profilée (21a) de l'organe de manoeuvre amovible (21) afin de pouvoir être solidarisée en rotation audit organe de manoeuvre (21) et qui comprend au moins un point de repérage indicatif de la position du boisseau, et, d'autre part, par des points de repère (22) imprimés ou gravés sur le boîtier (7), disposés en correspondance des différentes positions possibles des boisseaux.

3. Rampe de perfusion selon la revendication 2, caractérisée en ce que l'ouverture (16a) de la bague (16) présente un profil comprenant au moins une extrémité distincte de nature à constituer le ou les points de repère indicatifs de la position du boisseau, que comprend la bague (16).

4. Rampe de perfusion selon la revendication 3, caractérisée en ce que l'ouverture (16a) de la bague (16) présente un profil triangulaire, chaque angle du triangle correspondant à l'une des ouvertures du boisseau du robinet (3).

5. Rampe de perfusion selon l'une des revendications 1 à 4, caractérisée en ce que la partie mâle profilée (21a) de l'organe de manoeuvre (21) traverse l'ouverture (16a) de la bague (16) et vient s'engager dans la cavité femelle (20) du boisseau.

6. Rampe de perfusion selon l'une des revendications 1 à 4, caractérisée en ce que la bague (16) comprend un prolongement axial (16b) faisant saillie vers l'intérieur du boîtier (7), ce prolongement (16b) fermant l'ouverture (16a) de la bague (16) et ayant un profil mâle complémentaire de la cavité femelle (20) du boisseau, cavité (20) dans laquelle il est destiné à être engagé.

7. Rampe de perfusion selon l'une des revendications 1 à 6, caractérisée en ce qu'elle comprend des orifices (9) ménagés dans la face supérieure du boîtier (7).

8. Rampe de perfusion selon l'une des revendications 1 à 7, caractérisée en ce qu'elle comprend des organes de manoeuvre (21) ayant des tiges de longueur différente.

9. Rampe de perfusion selon l'une des revendications 1 à 8, caractérisée en ce qu'elle comprend une platine (10) pour son accrochage en hauteur, comprenant une partie concave (11) destinée à être engagée autour d'un mât ou d'une potence et une vis (12) pour le serrage dudit mât ou de ladite potence contre ladite partie concave (11).

## Claims

1. Infusion manifold, of the type having several tap cocks, enveloped in a flexible cellular material impregnated with an antiseptic liquid and contained in a casing, characterised in that each manoeuvring device (21) for the cocks (3) is removable and can be fixed in rotation to the head of the tap of a cock (3) by means of a profiled male part (16b, 21a) able to be engaged, through openings (15) formed in the casing (7) and in the cellular material (6) facing the taps of the cocks (3), in a female cavity (20) with a corresponding profile, means for indicating the position of the tap, visible from the outside of the casing (7) when the said manoeuvring device (21) is withdrawn, also being provided.

2. Infusion manifold according to Claim 1, characterised in that the indicator means consist, on the one hand, for each cock (3), of a ring (16) which is engaged on the opening comprised in the casing (7) facing the cock (3) and which is mounted so as to pivot with respect to the casing (7), whose opening (16a) has a profile complementary to that of the profiled male part (21a) of the removable manoeuvring device (21) so as to be able to be fixed in rotation to the said manoeuvring device (21) and which comprises at least one indicator point indicating the position of the tap, and, on the other hand, by reference points (22) printed or engraved on the casing (7), disposed so as to correspond to the various possible positions of the taps.

3. Infusion manifold according to Claim 2, characterised in that the opening (16a) in the ring (16) has a profile comprising at least one distinct end to form the reference point or points, indicating the position of the tap, that the ring (16) comprises.

4. Infusion manifold according to Claim 3, characterised in that the opening (16a) in the ring (16) has a triangular profile, each angle of the triangle corresponding to one of the openings in the tap cock (3).

5. Infusion manifold according to one of Claims 1 to 4, characterised in that the profiled male part (21a) of the manoeuvring device (21) passes through the opening (16a) in the ring (16) and is engaged in the female cavity (20) of the tap.

6. Infusion manifold according to one of Claims 1 to 4, characterised in that the ring (16) comprises an axial extension (16b) projecting towards the inside of the casing (7), this extension (16b) closing off the opening (16a) in the ring (16) and having a male profile complementary to the female cavity (20) of the tap, a cavity (20) in which it is designed to be engaged.

7. Infusion manifold according to one of Claims 1 to 6, characterised in that it comprises orifices (9) formed in the top face of the casing (7).

8. Infusion manifold according to one of Claims 1 to 7, characterised in that it comprises manoeuvring devices (21) having rods of different lengths.

9. Infusion manifold according to one of Claims 1 to 8, characterised in that it comprises a plate (10) for locking it at the required height, comprising a concave part (11) designed to be engaged about a pole or bracket and a screw (12) for clamping the said pole or bracket against the said concave part (11).

## Patentansprüche

1. Infusionsverteiler, mit mehreren Kükenhähnen, die von einem weichen, porösen, mit einer antiseptischen Flüssigkeit getränkten Material umhüllt und in einem Gehäuse enthalten sind, dadurch gekennzeichnet, daß jedes Betätigungsorgan (21) der Hähne (3) herausziehbar ist und durch ein profiliertes männliches Teil (16b, 21a) mit dem Kopf des Kükens eines Hahnes (3) drehbar verbunden werden kann, welches durch Öffnungen (15) in dem Gehäuse (7) und in dem porösen Material (6), die den Küken der Hähne (3) gegenüberliegend ausgebildete sind, in eine weibliche Vertiefung (20) entsprechenden Profils eingesteckt werden kann, und bei dem außerdem Mittel zum Anzeigen der Stellung des Kükens vorgesehen sind, die außen am Gehäuse (7) sichtbar sind, wenn das Betätigungsorgan (21) herausgezogen ist.

2. Infusionsverteiler nach Anspruch 1, dadurch gekennzeichnet, daß die Mittel zur Anzeige einerseits von einem Ring (16) für jeden Hahn (3) gebildet werden, der in die dem Hahn (3) gegenüberliegende Öffnung des Gehäuses (7) eingesetzt ist und der in bezug auf das Gehäuse (7) drehbar angeordnet ist, dessen Öffnung (16a) ein zu dem des profilierten männlichen Teils (21a) des herausziehbaren Betätigungsorgans (21) komplementäres Profil hat, um drehbar mit dem Betätigungsorgan (21) verbunden werden zu können, und der wenigstens eine Anzeigestelle aufweist, um die Position des Kükens anzuzeigen, und andererseits von auf dem Gehäuse (7) aufgedruckten oder eingravierten Anzeigestellen (22), die entsprechend der verschiedenen möglichen Positionen der Küken angeordnet sind.

3. Infusionsverteiler nach Anspruch 2, dadurch gekennzeichnet, daß die Öffnung (16a) des Rings (16) ein Profil aufweist, das wenigstens eine deutlich sichtbare Spitze hat, um so die eine oder die Anzeigestellen zu bilden, die die Position des Kükens anzeigen, die der Ring (16) einnehmen kann.

4. Infusionsverteiler nach Anspruch 3, dadurch gekennzeichnet, daß die Öffnung (16a) des Rings (16) ein dreieckiges Profil aufweist, wobei jeder Winkel des Dreiecks einer der Öffnungen des Kükens des Hahns (3) entspricht.

5. Infusionsverteiler nach einem der Ansprüche 1 bis 4, dadurch gekennzeichnet, daß das profilierte männliche Teil (21a) des Betätigungsorgans (21) die Öffnung (16a) des Rings (16) durchquert und in die weibliche Vertiefung (20) des Kükens eingesteckt ist.

6. Infusionsverteiler nach einem der Ansprüche 1 bis 4, dadurch gekennzeichnet, daß der Ring (16) eine axiale Verlängerung (16b) aufweist, die ins Innere des Gehäuses (7) übersteht, wobei diese Verlängerung (16b) die Öffnung (16a) des Rings (16) verschließt und ein zur weiblichen Vertiefung (20) des Kükens komplementäres männliches Profil hat, das zum Einstecken in die Vertiefung (20) bestimmt ist.

7. Infusionsverteiler nach einem der Ansprüche 1 bis 6, dadurch gekennzeichnet, daß er in der Oberseite des Gehäuses (7) ausgebildete Öffnungen (9) aufweist.

8. Infusionsverteiler nach einem der Ansprüche 1 bis 7, dadurch gekennzeichnet, daß er Betätigungsorgane (21) mit unterschiedlich langem Schaft aufweist.

9. Infusionsverteiler nach einem der Ansprüche 1 bis 8, dadurch gekennzeichnet, daß er eine Trageplatte (10) für seine erhöhte Anordnung aufweist, die einen konkaven Teil (11) aufweist, der dazu bestimmt ist, eine Stange oder einen Träger zu umgreifen, und die eine Schraube (12) aufweist, um die Stange oder den Träger gegen den konkaven Teil (11) zu drücken.
